# EUROPEAN PATENT APPLICATION

(11) **EP 4 722 689 A1**
(43) Date of publication of application: **08.04.2026**
(21) Application number: 24815272.0
(22) Date of filing: 20.05.2024
(51) Int. Cl.: G01N 5/02

(54) **IDENTIFICATION DEVICE, IDENTIFICATION SYSTEM, AND IDENTIFICATION METHOD**

(30) Priority: 01.06.2023 JP 2023091233
(71) Applicant: I-PEX Inc., Fushimi-ku, Kyoto-shi Kyoto 612-8024 (JP)
(72) Inventor: OGATA Kenji, Ogori-shi, Fukuoka 838-0106 (JP); SAKAMOTO Hisatoshi, Ogori-shi, Fukuoka 838-0106 (JP)
(74) Representative: SSM Sandmair
(86) International application number: PCT/JP2024/018531
(87) International publication number: WO 2024/247788

(57) **Abstract**

An identification device (2) includes detection elements (5) (D[1] to D[M], where M is a natural number of 2 or more) and an identifier (11). Each of the detection elements (5) reacts to one of substances of types different from one another among types of substances emitted from a generation source (T) and outputs a detection value having a magnitude corresponding to the amount of a substance to which the detection element (5) has reacted. The identifier (11) identifies, based on information indicating an order of the detection elements (5) when substances are detected by the detection elements (5) , a type of a generation source (T) of the substances with reference to information indicating a relationship between an order of the detection elements (5) that is ordered based on magnitudes of detection values when substances emitted from a generation source (T) of a known type are detected by the detection elements (5) and the type of the known generation source (T).

## Description

### Technical Field

The present disclosure relates to an identification device, an identification system, and an identification method.

### Background Art

In Patent Literature 1, a chemical sensor device that identifies a generation source, based on the amount of change in a resonant frequency of an oscillator that occurs when a substance is adsorbed or desorbed is disclosed. The chemical sensor device includes a plurality of oscillators that exhibits different adsorption/desorption characteristics for substances different from one another. Each oscillator is excited when AC voltage is applied. By comparing map data obtained by mapping resonance frequencies of a plurality of oscillators which predetermined substances are adsorbed on or desorbed from with map data obtained by mapping detected resonance frequencies, the substances are identified.

### Citation List

### Patent Literature

Patent Literature 1: Unexamined Japanese Patent Application Publication No. 2009-204584

### Summary of Invention

### Technical Problem

The above-described chemical sensor device is susceptible to influence of noise of equipment and changes in temperature and humidity during detection. Since due to differences in temperature and humidity in the surroundings at the time of detection, the map data may shift even for the same generation source, the chemical sensor device has had a problem in that it is difficult to stabilize accuracy of identification of a generation source.

The present disclosure has been made in consideration of the above-described circumstances, and an objective of the present disclosure is to provide an identification device, an identification system, and an identification method capable of stabilizing accuracy of identification of a generation source.

### Solution to Problem

To achieve the above-described objective, an identification device according to a first aspect of the present disclosure includes:
a plurality of detection elements each of which reacts to one of substances of types different from one another among a plurality of types of substances emitted from a generation source and outputs a detection value having a magnitude corresponding to an amount of a substance to which the detection element has reacted; and
an identifier that identifies, based on information indicating an order of the plurality of detection elements when substances are detected by the plurality of detection elements, characteristic information of a generation source of the substances with reference to information indicating a relationship between an order of the plurality of detection elements, the order being ordered based on magnitudes of detection values detected by the plurality of detection elements when substances emitted from a generation source the characteristic information of which indicating a type or a state is known are detected by the plurality of detection elements, and the characteristic information of the known generation source.

In the substances, a substance causing an odor to be sensed may be included.

An identification system according to a second aspect of the present disclosure includes:
a plurality of detection elements each of which reacts to one of substances of types different from one another among a plurality of types of substances emitted from a generation source and outputs a detection value having a magnitude corresponding to an amount of a substance to which the detection element has reacted;
a generator that generates information indicating orders of the plurality of detection elements, each of the orders being ordered based on magnitudes of detection values when detection is performed by the plurality of detection elements with respect to one of a plurality of known generation sources pieces of characteristic information of which indicating types or states are different from one another, in association with the pieces of characteristic information of the known generation sources; and
an identifier that identifies, based on information indicating an order of the plurality of detection elements when substances are detected by the plurality of detection elements, the characteristic information of a generation source of the substances with reference to information indicating relationships between orders of the plurality of detection elements and the pieces of characteristic information of the known generation sources, the information being generated by the generator.

In a case where when detection using the plurality of detection elements is performed multiple times with respect to known generation sources the pieces of characteristic information of which are same, there exist a detection element a position of which in an order of magnitudes of detection values changes and, even when the detection element is excluded, orders of remaining detection elements do not overlap each other between the generation sources, the generator may narrow down the detection elements to the remaining detection elements and generate information indicating an order of the detection elements, and the identifier may identify, based on information indicating an order of the detection elements when substances are detected by the detection elements that are narrowed down by the generator, the characteristic information of a generation source of the substances.

In a case where when detection using the plurality of detection elements is performed multiple times with respect to known generation sources the pieces of characteristic information of which are the same, there exist detection elements positions of which in an order of magnitudes of detection values change and, even when the detection elements are grouped, orders do not overlap each other between generation sources, the generator may group detection elements the positions of which in an order are consecutive among detection elements the positions of which in an order change and generate information indicating an order of groups of the detection elements, and the identifier may identify, based on information indicating an order of the detection elements when substances are detected by the detection elements in groups generated by the generator, the characteristic information of a generation source of the substances.

The generator may group generation sources with respect to which orders of the detection elements are same when detection using the plurality of detection elements is performed with respect to a plurality of known generation sources the pieces of characteristic information of which are different from one another and generate information indicating an order of the detection elements in association with a group, and the identifier may identify, based on information indicating an order of the plurality of detection elements when substances are detected by the plurality of detection elements, the characteristic information of a group of generation sources of the substances with reference to information indicating a relationship between an order of the plurality of detection elements and a group of the known generation sources, the information being generated by the generator.

The generator may generate information indicating detection values when detection is performed by the plurality of detection elements with respect to each of a plurality of known generation sources the pieces of characteristic information of which are different from one another in association with the pieces of characteristic information of the known generation sources, and the identifier may identify, based on, in conjunction with information indicating an order of the detection elements when substances are detected by the plurality of detection elements, detection values detected by the detection elements when substances are detected by the plurality of detection elements, the characteristic information of a generation source of the substances with reference to, in conjunction with information indicating a relationship between an order of the detection elements and the characteristic information of the known generation source, information indicating a relationship between detection values detected by the detection elements and the characteristic information of the known generation source, the information being generated by the generator.

An identification method according to a third aspect of the present disclosure is executed by an identification device that identifies a generation source, using a plurality of detection elements each of which reacts to one of substances of types different from one another among a plurality of types of substances emitted from a generation source and outputs a detection value having a magnitude corresponding to an amount of a substance to which the detection element has reacted, the identification method including
identifying, based on information indicating an order of the plurality of detection elements when substances are detected by the plurality of detection elements, the characteristic information of a generation source of the substances with reference to information indicating relationships between orders of the plurality of detection elements, the orders being ordered based on magnitudes of detection values detected by the plurality of detection elements when substances emitted from known generation sources pieces of characteristic information of which indicating types or states are different from one another are detected by the plurality of detection elements, and the pieces of characteristic information of the known generation sources.

### Advantageous Effects of Invention

According to the present disclosure, it is possible to stabilize accuracy of identification of a generation source.

### Brief Description of Drawings

FIG. 1 is a block diagram illustrating a configuration of an identification system according to Embodiment 1 of the present disclosure;
FIG. 2A is a graph illustrating an example of map data of detection values detected by a plurality of detection elements;
FIG. 2B is a table illustrating an order of magnitudes of detection values detected by the plurality of detection elements;
FIG. 3 is a block diagram illustrating a configuration for the identification system in FIG. 1 to perform identification;
FIG. 4 is a block diagram illustrating a configuration for the identification system in FIG. 1 to perform registration of an order;
FIG. 5 is a block diagram illustrating a hardware configuration of an identifier in FIG. 1;
FIG. 6 is a flowchart of registration mode processing;
FIG. 7 is a flowchart of identification mode processing;
FIG. 8 is a flowchart of registration mode processing of an identification system according to Embodiment 2 of the present disclosure;
FIG. 9A is a diagram illustrating a first example of a case where positions of detection elements in an order change;
FIG. 9B is a diagram illustrating a second example of the case where the positions of detection elements in an order change;
FIG. 10 is a flowchart of registration mode processing of an identification system according to Embodiment 3 of the present disclosure;
FIG. 11A is a diagram illustrating a first example of grouping of detection elements;
FIG. 11B is a diagram illustrating a second example of the grouping of detection elements;
FIG. 12 is a flowchart of registration mode processing of an identification system according to Embodiment 4 of the present disclosure;
FIG. 13A is a diagram illustrating a first example of grouping of generation sources;
FIG. 13B is a diagram illustrating a second example of the grouping of generation sources;
FIG. 14 is a block diagram illustrating a configuration of an identification system according to Embodiment 5 of the present disclosure;
FIG. 15 is a flowchart of registration mode processing of the identification system in FIG. 14;
FIG. 16 is a flowchart of identification mode processing of the identification system in FIG. 14;
FIG. 17 is a block diagram illustrating a configuration of an identification system according to Embodiment 6 of the present disclosure;
FIG. 18A is a table illustrating orders of magnitudes of detection values detected by a plurality of detection elements from two generation sources the types of which are different from each other;
FIG. 18B is a graph illustrating map data of detection values detected by a plurality of detection elements from the two generation sources the types of which are different from each other;
FIG. 19A is a schematic diagram illustrating an example of a detector; and
FIG. 19B is a perspective view illustrating detection elements with a portion of the detector cut off.

### Description of Embodiments

Embodiments of the present disclosure are described below in detail with reference to the drawings. In the drawings, the same or equivalent parts are designated by the same reference numerals. An identification device, an identification system, and an identification method according to the present embodiment identify a generation source of substances.

### Embodiment 1

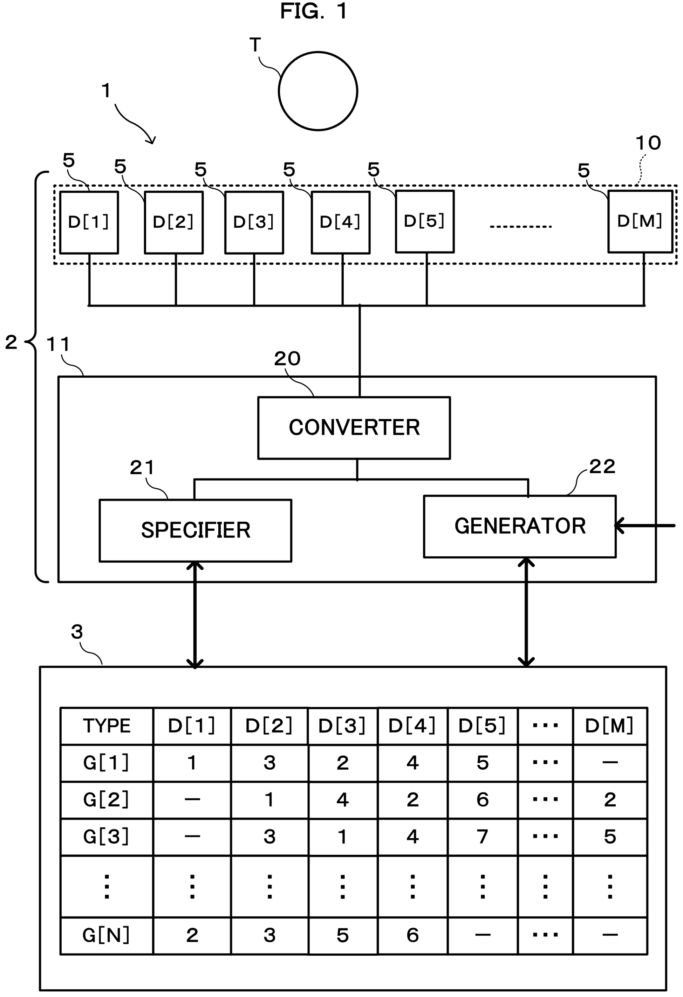

Embodiment 1 of the present disclosure is described below. As illustrated in FIG. 1, an identification system 1 according to the present embodiment identifies, as characteristic information of a generation source T of substances, a type of the generation source T. The identification system 1 includes an identification device 2 and a storage device 3. The identification device 2 includes a detector 10 and an identifier 11.

### Detector

The detector 10 includes a plurality of detection elements 5. Each of the plurality of detection elements 5 reacts to one of substances of types different from one another among a plurality of types of substances emitted from a generation source T. Each detection element 5 outputs a detection value having a magnitude corresponding to the amount of the substance to which the detection element 5 has reacted. For example, each of the detection elements 5 may be a detection element that includes an oscillator having a substance adsorption film that adsorbs a substance. When a substance is adsorbed on the substance adsorption film, total weight of the oscillator increases and a vibration level changes. Magnitude of change in the vibration level corresponds to the amount of adsorption of the substance on the substance adsorption film. Each of the detection elements 5 outputs the magnitude of change in the vibration level as the amount of adsorption of the substance, that is, a reaction level (detection value) that indicates a magnitude corresponding to the amount of reacted components.

Each of the detection elements 5 is assigned a unique identification number. Hereinafter, identification numbers assigned to the individual detection elements 5 are denoted by D[1] to D[M] (M is a natural number of 2 or more). In the present embodiment, it is assumed that M is a natural number of 6 or more. Each of the detection elements 5 outputs data including a detection value with which an identification number is associated. This configuration of the data enables by which detection element 5 a detection value included in data is detected to be grasped.

### Identifier

The identifier 11 accepts, as input, data including detection values and identification numbers output from the plurality of detection elements 5 in the detector 10, and performs information processing on the input data. The identifier 11 includes a converter 20, a specifier 21, and a generator 22.

### Converter

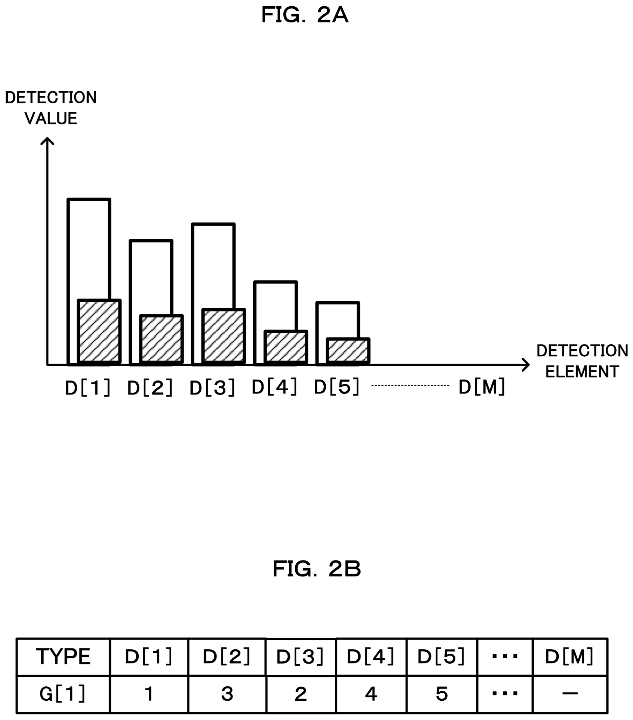

The converter 20 generates, based on data including detection values and identification numbers output from the plurality of detection elements 5, information indicating an order of the plurality of detection elements 5 (D[1] to D[M]) when substances are detected by the plurality of detection elements 5 (D[1] to D[M]). In other words, the converter 20 converts map data of detection values output from the plurality of detection elements 5 into information indicating an order of magnitudes of the detection values. For example, in a case where when the magnitudes of detection values detected by the detection elements 5 (D[1] to D[M]) are expressed as map data, the map data are presented as a white bar graph in FIG. 2A, the converter 20 generates information indicating an order of the plurality of detection elements 5 (D[1] to D[M]) as illustrated in FIG. 2B. Note that a detection element 5 that outputs a detection value lower than a threshold value may be excluded from the order. Note that the order may be an order ordered based on detection values normalized by a reference value such as a maximum value among the detection values detected by the detection elements 5, or may be an order ordered based on the detection values themselves detected by the detection elements 5.

In FIG. 2A, two patterns of detection values by the detection elements 5 that are detected for the same generation source T are illustrated by a white bar graph and a hatched bar graph. As illustrated in FIG. 2A, while even from the same generation source T, the magnitude of a detection value detected by each detection element 5 varies depending on a measurement condition and the like, the order of the plurality of detection elements 5 (D[1] to D[M]) based on the magnitudes of detection values remains invariable.

### Specifier

The specifier 21 identifies characteristic information (type) of a generation source T, based on information indicating an order of the plurality of detection elements 5 that is ordered by magnitudes of detection values when substances are detected and that is generated by the converter 20. In the storage device 3, information indicating relationships between orders of the magnitudes of detection values detected by the plurality of detection elements 5 and types of known generation sources T is stored. The specifier 21, with reference to the information indicating relationships between orders of the plurality of detection elements 5 and types of known generation sources T stored in the storage device 3, identifies a type of a generation source T of the substances.

In FIG. 1, in the storage device 3, orders of the detection elements 5 (D[1] to D[M]) are stored with respect to generation sources T (G[1] to G[N], where N is a natural number of 2 or more) the types of which are known in association with the types of the known generation source T (G[1] to G[N]). In the present embodiment, N is a natural number of 4 or more. There is no particular restriction on the generation sources serving as G[1] to G[N], and, for example, various objects that emit substances into the air, such as fruits like an apple, a banana, and a tangerine and vegetables, can be specified as a generation source. For example, in the storage device 3, each of G[1], G[2], G[3], ... , and G[N] is stored as a generation source T the type of which is known in association with an order of the plurality of detection elements 5. For example, the order of the detection elements 5 that is associated with G[1] is (D[1], D[2], D[3], D[4], D[5]) = (1, 3, 2, 4, 5).

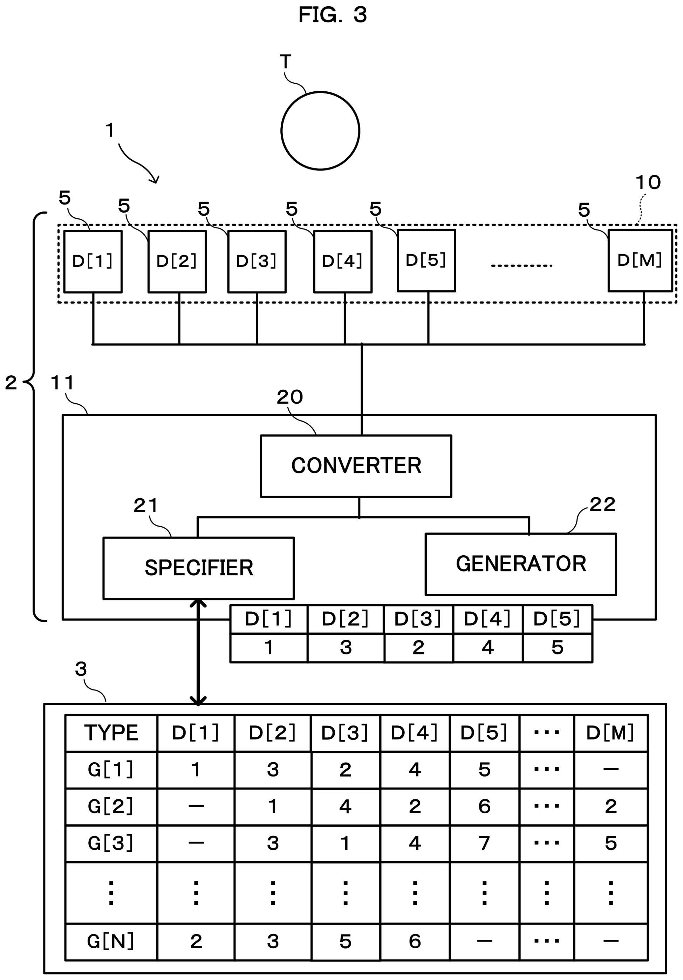

It is now assumed that detection by the plurality of detection elements 5 is performed on a generation source T the type of which is unknown and information indicating an order of magnitudes of detection values detected by the plurality of detection elements 5 is generated by the converter 20. The specifier 21 matches the orders of the plurality of detection elements 5 stored in storage device 3 with the order of the plurality of detection elements 5 for the unknown generation source T and specifies, as the unknown generation source T, a generation source T associated with an order of the plurality of detection elements 5 that coincides with the order of the plurality of detection element 5 of the unknown generation source T. For example, as illustrated in FIG. 3, when the order of the detection elements 5 of the unknown generation source T is (D[1], D[2], D[3], D[4], D[5]) = (1, 3, 2, 4, 5), the unknown generation source T is specified as G[1].

### Generator

To perform the specification by the specifier 21, it is required to store, in the storage device 3, information indicating relationships between orders of the plurality of detection elements 5 and types of known generation sources T. The generator 22 generates the information that the storage device 3 stores.

The generator 22 accepts, as input, information indicating a type of a generation source T of substances detected by the plurality of detection elements 5. The input is performed through operation input by a user. For example, prior to substances emitted from the generation source T (G[1]) being detected by the plurality of detection elements 5, the type of the generation source T being G[1] is input.

The generator 22 stores information indicating an order of magnitudes of detection values of the plurality of detection elements 5 that is detected by the plurality of detection elements 5 with respect to a generation source T the type of which is input and that is generated by the converter 20 and the input type of the generation source T in association with each other in the storage device 3.

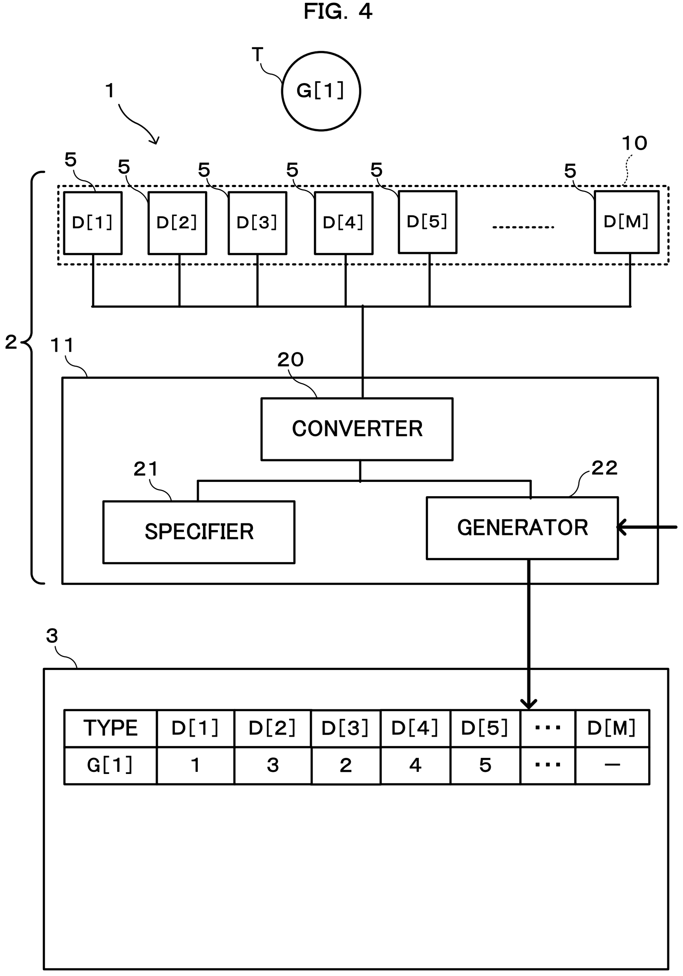

For example, as illustrated in FIG. 4, when the type G[1] of the generation source T is input and the plurality of detection elements 5 detects substances emitted from the generation source T (type G[1]), the converter 20 generates information indicating an order of the plurality of detection elements 5 that is ordered based on the magnitudes of detection values detected by the plurality of detection elements 5 when the substances emitted from the generation source T (G[1]) are detected. The generator 22 generates the information indicating the order of the plurality of detection elements in association with the known generation source G[1] and stores the generated information in the storage device 3.

Likewise, with respect to G[2], G[3], G[4], ... , and G[N] as generation sources T the types of which are known, the substance detection performed by the plurality of detection elements 5, the generation of order performed by the converter 20, and the registration of the order performed by the generator 22 are also performed. Through this processing, in the storage device 3, information indicating orders of the plurality of detection elements 5 each of which is associated with one of G[1], G[2], G[3], G[4], ... , and G[N], which are the generation sources T the types of which are known, are stored.

### Hardware Configuration of Identifier

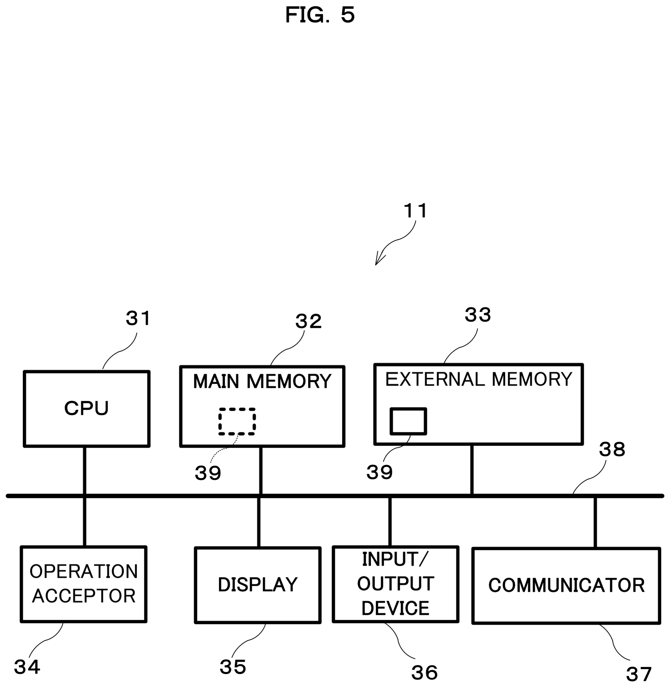

The identifier 11 illustrated in FIG. 1 is achieved by, for example, a computer with a hardware configuration illustrated in FIG. 5 executing a software program. Specifically, the identifier 11 includes a central processing unit (CPU) 31 that controls the overall device, a main memory 32 that operates as a working area or the like for the CPU 31, an external memory 33 that stores an operation program or the like of the CPU 31, an operation acceptor 34, a display 35, an input/output device 36, a communicator 37, and an internal bus 38 connecting the foregoing to one another.

The main memory 32 includes a random access memory (RAM) and the like. Into the main memory 32, a program 39 to be executed by the CPU 31 is loaded from the external memory 33. In addition, the main memory 32 is also used as a working area (temporary storage area of data) for the CPU 31.

The external memory 33 includes a nonvolatile memory, such as a flash memory and a hard disk. In the external memory 33, the program 39 to be executed by the CPU 31 is stored in advance.

The operation acceptor 34 includes devices such as a keyboard and a mouse, and an interface device that connects the devices to the internal bus 38.

The display 35 includes a device for display, such as a cathode ray tube (CRT) and a liquid crystal monitor.

The input/output device 36 is an interface that performs transmission and reception of data with an external device. The input/output device 36, in accordance with a command from the CPU 31, outputs a detection command signal to the detection elements 5 and also inputs detection signals from the detection elements 5. An input detection signal is stored in the main memory 32 or the external memory 33, or displayed on the display 35.

The communicator 37 is an interface for data communication. Data communication with an external device can be performed via the communicator 37. For example, when the storage device 3 is configured as an external device, writing and reading of data to and from the storage device 3 are performed through data communication via the communicator 37.

Functions of the identifier 11 can be implemented in a computer system including one or more computers each of which includes one or more processors and one or more storage devices including a non-transitory storage medium. A plurality of computers, while performing communication via communication networks connected to one another, achieves the function of the identifier 11. For example, some of the functions of the identifier 11 may be implemented in one computer, while the other functions may be implemented in another computer.

Next, operation of the identification system 1 according to the present embodiment is described. Through this operation, the identification method is executed. The identification system 1 operates in one of two operation modes. The first operating mode is a registration mode in which data for identifying a type of a generation source T are generated and stored in the storage device 3. The second operating mode is an identification mode in which, with reference to the data stored in the storage device 3, the type of a generation source T is identified based on a detection result detected by the plurality of detection elements 5.

### Registration Mode Processing

First, processing of the registration mode (registration mode processing) is described. In the registration mode, m is used as a variable. The variable m takes a value from 1 to M (M is a natural number of 2 or more). This value indicates a generation source T the type of which is known that is to be identified at this time.

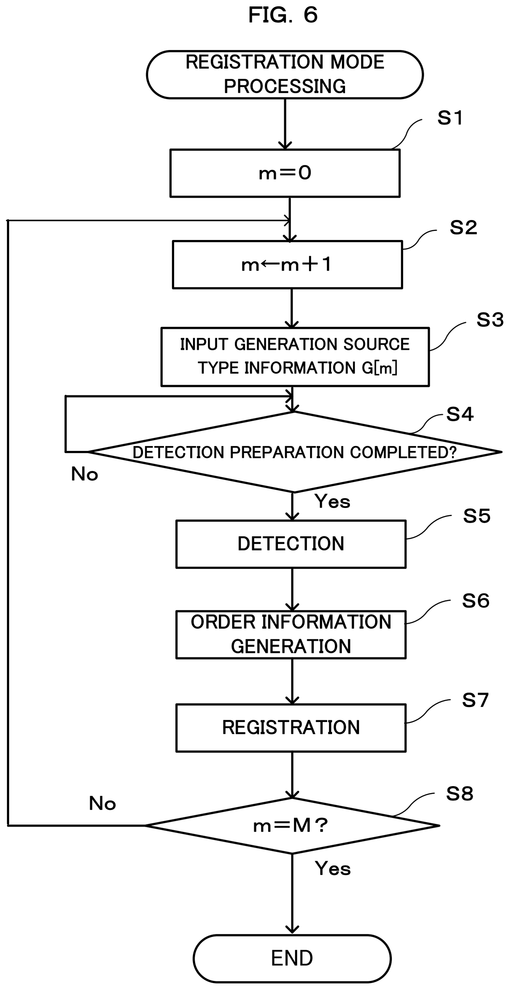

In the registration mode processing, first, the identifier 11 initializes the variable m to 0 (step S1) and increments the variable m by 1 (step S2), as illustrated in FIG. 6.

Next, the generator 22 constituting the identifier 11 accepts, as input, information about the type of the generation source T (step S3). In this step, for example, the type of the generation source T being G[1] is input. In this step, for example, "apple" is registered as G[1].

Next, the identifier 11 waits until a state in which a generation source T the type of which is known can be detected is established, that is, until detection preparation is completed (step S4; No). Specifically, the detection preparation is considered to be completed at a time point when after the plurality of detection elements 5 in the detector 10 is brought into a state in which each of the detection elements 5 is not reacting to a substance to be identified by the detection element 5, that is, a state in which the detection elements 5 are initialized, the generation source T of the type input in step S3 (G[1] when m = 1) is placed in a vicinity of the detector 10 and substances emitted from the generation source T has become detectable by the plurality of detection elements 5. In the present embodiment, when the detector 10 is in a state of waiting for input of an operation to the generator 22 by the user and an operation input indicating completion of preparation is performed, it is determined that the detection preparation is completed.

When the detection preparation is completed (step S4; Yes), the identifier 11 causes the plurality of detection elements 5 to detect substances emitted from the generation source T the type of which is known (step S5). Through this processing, data including detection values of substances emitted from the generation source T the type of which is known and identification information are sent from the detection elements 5 to the converter 20 of the identifier 11.

When the data are input from the detection elements 5, the converter 20 of the identifier 11 generates information indicating an order of the plurality of detection elements 5, based on magnitudes of the detection values output from the plurality of detection elements 5 (step S6). Through this processing, information indicating an order of the plurality of detection elements 5, such as D[1], D[3], D[2], D[4], and D[5] in descending order of the detection values, is generated with respect to the generation source T (type G[1]), as illustrated in, for example, FIG. 4.

Next, the generator 22 of the identifier 11 registers the order generated by the converter 20 and the input information indicating the type of the generation source T in association with each other in the storage device 3 (step S7).

Next, the identifier 11 determines whether or not the variable m is M (step S8). When the variable m is not M (step S8; No), the identifier 11 returns to step S2. Subsequently, the increment of the variable m (step S2; m = 2), the input of information about the generation source G[2] (step S3), the waiting for completion of detection preparation including initialization of the detection elements 5 (step S4), the detection of substances emitted from the generation source G[2] by the plurality of detection elements 5 (step S5), the order information generation (step S6), the registration (step S7), and the determination of the variable m (step S8) are performed. Further, for m = 3 to M, steps S2 to S8 are performed, in which orders of the plurality of detection elements 5 are generated and pieces of information indicating the orders are stored in the storage device 3 in association with the types G[3] to G[M] of the generation sources T. When the variable m is determined to be M (step S8; Yes), the identifier 11 terminates the registration mode processing.

### Identification Mode

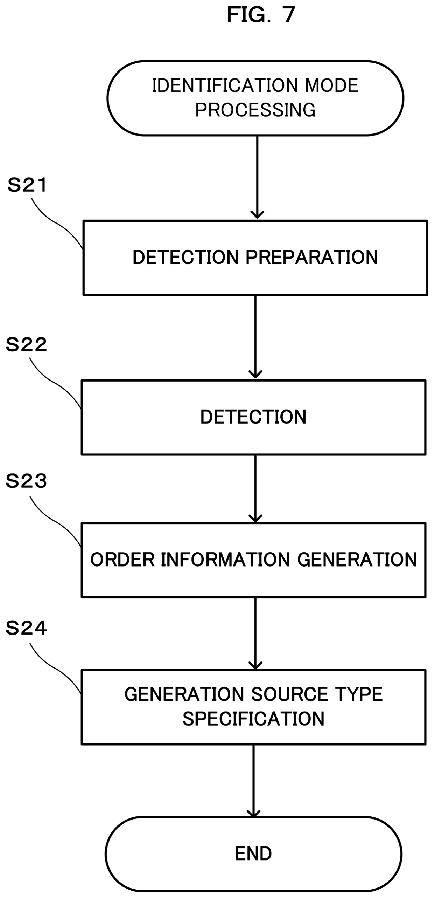

Next, processing of the identification mode (identification mode processing) is described. In the identification mode processing, first, the identifier 11, after initializing the plurality of detection elements 5, performs detection preparation (step S21), as illustrated in FIG. 7. Then, the detector 10 is brought close to a generation source T the type of which is unknown that is to be identified. Next, the identifier 11 causes the plurality of detection elements 5 to detect substances emitted from the generation source T to be identified (step S22). Each of the plurality of detection elements 5 sends data including a detection value and the identification number of the detection element 5 to the identifier 11.

Next, the converter 20 of the identifier 11 generates information indicating an order of the plurality of detection elements 5, based on magnitudes of detection values output from the plurality of detection elements 5 (step S23). Through this processing, for example, information indicating the order of the plurality of detection elements 5, such as D[1], D[3], D[2], D[4], and D[5] in descending order of the detection values, is generated, as illustrated in FIG. 3.

Next, the specifier 21 matches the order generated by the converter 20 with the orders stored in the storage device 3, retrieves the type of a generation source T associated with an order that coincides with the order generated by the converter 20, and specifies the generation source T (step S24). For example, G[1] is specified as the generation source T associated with the order D[1], D[3], D[2], D[4], and D[5] in descending order of the magnitudes of the detection values, as illustrated in FIG. 3. Subsequently, the identifier 11 terminates the identification mode processing.

As described in detail in the foregoing, according to the identification system 1 according to the present embodiment, the type of a generation source T of substances is specified not by the magnitudes of detection values (reaction levels) of the plurality of detection elements 5, which are prone to error, but by the order of the magnitudes of the detection values. Since when configured in such a manner, the type of a generation source T can be identified using information that is less likely to contain error, accuracy of identification of the type of a generation source T can be stabilized.

It is possible to identify a generation source T through matching of map data of magnitudes of detection values detected by the plurality of detection elements 5. However, map data of magnitudes of detection values detected by the detection elements 5 not only differ every time the detection is performed, as illustrated in, for example, FIG. 2A but also include influence of and error due to temperature and humidity. Since in the identification system 1 according to the present embodiment, the amount of information used for specification is reduced and a generation source T is identified using an order as a criterion, the accuracy of the identification can be improved.

Note that an order of the plurality of detection elements 5 may include detection elements 5 having the same position in the order due to detection values detected by the detection elements 5 being the same.

### Embodiment 2

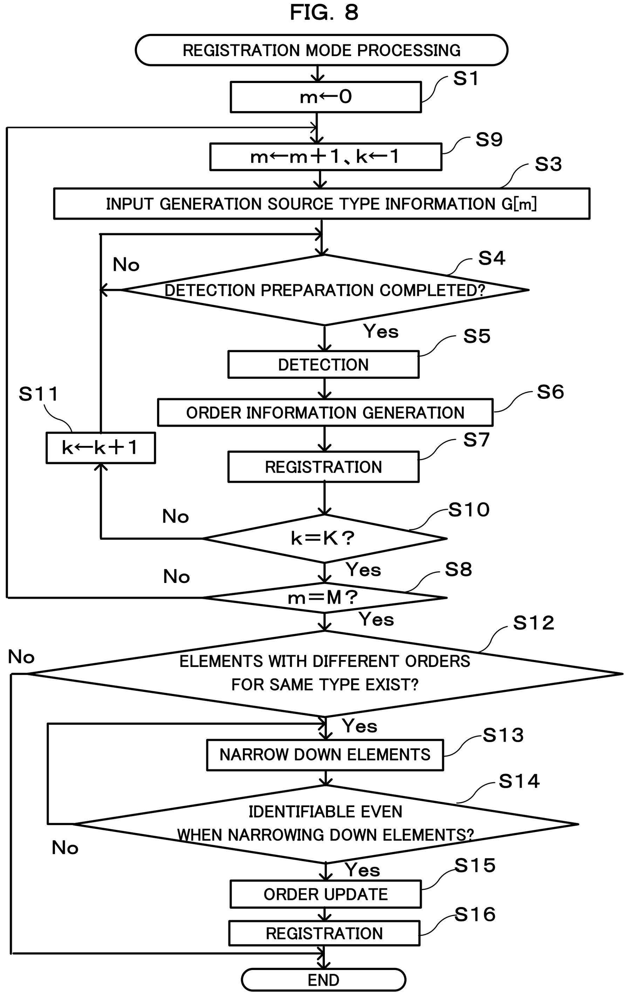

Next, Embodiment 2 of the present disclosure is described. A configuration of an identification system 1 according to the present embodiment is the same as the configuration of the identification system 1 according to Embodiment 1 described above illustrated in FIG. 1. Operation, in particular registration mode processing, of an identifier 11 of the identification system 1 according to the present embodiment is different from that of the identification system 1 according to Embodiment 1 described above (see FIG. 6), as illustrated in FIG. 8. In the registration mode processing according to the present embodiment, narrowing down of detection elements 5 is performed.

### Registration Mode Processing

The registration mode processing that is executed by the identifier 11 constituting an identification device 2 according to the present embodiment is described. In the registration mode processing, a variable k is used in addition to a variable m. The variable m is the same as that in Embodiment 1 described above in that the variable m takes a value from 1 to M and serves as a value indicating a type of a known generation source T. The variable k takes a value from 1 to K (K is a natural number of 2 or more). The value taken by the variable k is a value indicating the number of times of detection with respect to generation sources T the types of which are the same. As the maximum number K of k, a value corresponding to the number of samples that is large enough to provide sufficient samples from a statistical point of view can be set.

In the registration mode processing, first, the identifier 11 initializes the variable m to 0 (step S1), and increments the variable m by 1 and initializes the variable k to 1 (step S9), as illustrated in FIG. 8.

Next, a generator 22 constituting the identifier 11 accepts, as input, information about a type of a generation source T (step S3). In this step, for example, the generation source T being G[1] is input. Next, the identifier 11 waits until a state in which substances from the generation source T the type of which is known can be detected is established, that is, until detection preparation is completed (step S4; No). A user, after initializing the plurality of detection elements 5, brings a detector 10 close to a vicinity of the generation source T to be identified and completes the detection preparation.

When the detection preparation is completed (step S4; Yes), the identifier 11 causes the plurality of detection elements 5 to detect substances emitted from the generation source T the type of which is known (step S5). Through this processing, data including detection values of substances emitted from the generation source T the type is known and identification information are sent from the detection elements 5 to a converter 20 of the identifier 11.

When the data are input from the detection elements 5, the converter 20 of the identifier 11 generates information indicating an order of the plurality of detection elements 5, based on magnitudes of the detection values output from the plurality of detection elements 5 (step S6).

Next, the generator 22 of the identifier 11 registers the order generated by the converter 20 and the input information indicating the type of the generation source T in association with each other in the storage device 3 (step S7).

Next, the identifier 11 determines whether or not the variable k is K (step S10). Since at this time, it still holds that k = 1, the determination results in negation, and the identifier 11 increments the variable k by 1 (step S11). The identifier 11 performs again the detection (step S5), the order information generation (step S6), and the registration (step S7), determines, with respect to the variable k, whether or not k = K holds (step S10), and, when the determination results in negation (step S10; No), performs the increment of the variable k (step S11). Through this processing, with respect to generation sources T of the same type (G[1]), the detection of reaction levels by the plurality of detection elements 5 is performed K times.

Note that, each time the detection in each detection cycle is performed, in step S4, desorbing operation of substances is performed on the plurality of detection elements 5 and initialization of the plurality of detection elements 5 is performed. When the detection elements 5 have oscillators, the desorption of substances is performed by vibrating the oscillators.

When with respect to the variable k, k = K holds (step S10; Yes), the identifier 11 determines whether or not the variable m is M (step S8). When the variable m is not M (step S8; No), the identifier 11 returns to step S9. Subsequently, the increment of the variables m and k and the initialization of the variable to 1 (step S9), the input of information about the generation source T (type: G[2]) (step S3), the waiting for completion of the detection preparation (step S4), the detection (step S5), the order information generation (step S6), the registration (step S7), the determination of the variable k (step S10), and the increment of the variable k (step S11) are repeated, and substance detection with respect to the generation source G[2] is performed K times. When k = K holds (step S10; Yes), the determination of the variable m (step S8) is performed, and further, for each of m = 3 to M, the substance detection is performed K times where each time the substance detection is performed, an order of the plurality of detection elements 5 is generated, as a result of which pieces of information indicating the orders are stored (registered) in the storage device 3 in association with the types G[3] to G[M] of the generation sources T.

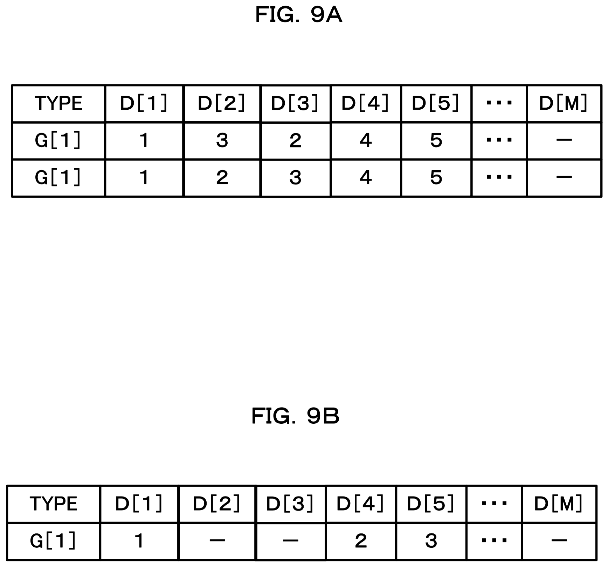

When subsequently, it is determined that the variable m is M (step S8; Yes), the generator 22 of the identifier 11 determines whether or not there exist detection elements 5 the positions of which in the orders are different between generation sources T the types of which are the same (step S12). For example, when, as illustrated in FIG. 9A, there exist detection elements 5 the positions of which in orders of the plurality of detection elements 5 are different between generation sources T the types of which are the same (G[1]), the determination in step S12 results in affirmation. Note that a case where positions of a plurality of detection elements 5 in orders that are different from each other change to the same position is included in the above-described affirmative case.

When there exist no detection elements 5 the positions of which in orders of the plurality of detection elements 5 are different between the generation sources T the types of which are the same (G[1]) (step S12; No), the identifier 11 terminates the registration mode processing.

In contrast, when there exist detection elements 5 the positions of which in orders of the plurality of detection elements 5 are different between the generation sources T the types of which are the same (G[1]) (step S12; Yes), the generator 22 narrows down the detection elements 5 (step S13). In this processing, the generator 22 excludes detection elements 5 the positions of which in the orders are different.

The generator 22 of the identifier 11 determines whether or not, even when detection elements 5 the positions of which in the orders are different are excluded from the orders of the plurality of detection elements 5, orders of the plurality of detection elements 5 corresponding to the generation sources T (G[1] to G[M]) differ from one another and each generation source T can be discriminated from the other generation sources T, that is, whether or not orders of the plurality of detection elements 5 that are the same as each other between generation sources T do not exist (step S14). When no orders of the plurality of detection elements 5 that are the same as each other exist with respect to the generation sources T (G[1] to G[M]) (step S14; Yes), the generator 22 updates information indicating orders of narrowed-down detection elements 5 (step S15) and registers, in the storage device 3, the orders generated by the converter 20 and the input information indicating the types of the generation sources T in association with each other (step S16). For example, when, as illustrated in FIG. 9A, positions of a detection element 5 (D[2]) and a detection element 5 (D[3]) in the orders are different, the generator 22 excludes the detection element 5 (D[2]) and the detection element 5 (D[3]) from the orders of the plurality of detection elements 5, as illustrated in FIG. 9B. Subsequently, the identifier 11 terminates the registration mode processing.

In contrast, when orders of the plurality of detection elements 5 that are the same with respect to the generation sources T (G[1] to G[M]) exist (step S14; No), the generator 22 narrows down the detection elements 5 (step S13). For example, when orders of narrowed-down detection elements 5 are the same with respect to G[1], G[2], and G[3], the generator 22 individually extracts a combination of detection elements 5 that makes a percentage of correct identifications of G[1] and G[2] greater than or equal to a threshold value from the narrowed-down detection elements 5. Further, the generator 22 individually extracts a combination of detection elements 5 that makes a percentage of correct identifications of G[2] and G[3] greater than or equal to the threshold value. Through this processing, as a combination of detection elements 5 to identify G[1] and G[2], for example, D[1], D[4], and D[5] are extracted and finally ordered. In addition, as a combination of detection elements 5 to identify G[2] and G[3], for example, D[1], D[7], and D[8] are extracted and finally ordered.

After the processing in step S13 is completed, the generator 22 performs steps S14, S15, and S16, and the identifier 11 terminates the registration mode processing.

Although as described above, when orders of the detection elements 5 that are the same between different generation sources T exist (step S12; No), the generator 22 narrows down the detection elements 5 (step S13), the narrowing down may be repeated by changing the threshold value for the percentage of correct identifications by which the narrowing down of the detection elements 5 is performed until orders of the detection elements 5 that are the same between different generation sources T are eliminated (step S13; Yes).

As described above, in the present embodiment, in the case where when detection using the plurality of detection elements 5 is performed multiple times with respect to known generation sources the types of which are the same, there exist detection elements 5 the positions of which in the orders of magnitudes of detection values change and, even when the detection elements 5 are excluded, orders of remaining detection elements 5 do not overlap each other between the generation sources T, the generator 22 narrows down the detection elements 5 to the remaining detection elements 5 and generates information indicating orders of the remaining detection elements 5.

A specifier 21 of the identifier 11 identifies, based on information indicating an order of the detection elements 5 when substances are detected by the detection elements 5 narrowed down by the generator 22, a type of a generation source T of the substances. Identification mode processing performed by the identifier 11 is the same as the identification mode processing performed by the identification system 1 according to Embodiment 1 described above (see FIG. 7). That is, detection preparation is performed with respect to a generation source T the type of which is unknown (step S21), detection of substances by the plurality of detection elements 5 is performed (step S22), order information is generated (step S23), and through matching of the order information, the generation source T is specified (step S24).

As described in the foregoing, according to the identification system 1 according to the present embodiment, in the case where the detection is performed multiple times with respect to generation sources T the types of which are the same, there exist detection elements 5 the positions of which in the orders are different between the generation sources T, and even when the detection elements 5 the positions of which in the orders are different are excluded, orders of the detection elements 5 are different with respect to all generation sources T and the generation sources T can be identified, the detection elements 5 the positions of which in the orders are different are excluded from the detection elements 5 used for the identification. Because of this configuration, it becomes possible to reduce the number of detection elements 5 to be actually used for the identification. Since as a result, the identifier 11 identifies, based on information indicating an order of the detection elements 5 when substances are detected by the detection elements 5 narrowed down by the generator 22, a type of the generation source T of the substances, the number of detection elements 5 can be reduced.

### Embodiment 3

Next, Embodiment 3 of the present disclosure is described. A configuration of an identification system 1 according to the present embodiment is the same as the configuration of the identification system 1 according to Embodiments 1 and 2 described above illustrated in FIG. 1. Operation, in particular registration mode processing, of an identifier 11 of the identification system 1 according to the present embodiment is different from that of the identification system 1 according to Embodiment 2 described above (see FIG. 8). In the registration mode processing executed by the identifier 11 according to the present embodiment, grouping of a plurality of detection elements 5 is performed.

### Registration Mode Processing

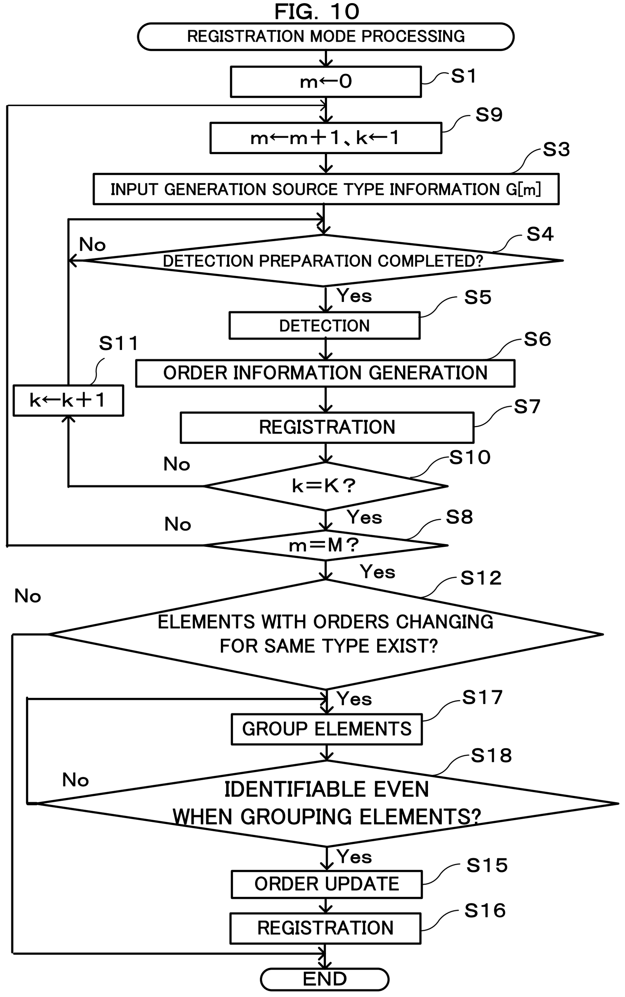

Since as illustrated in FIG. 10, in the registration mode processing, a process flow in steps S1, S9, S3 to S7, S10, S11, and S8 is the same as that in the registration mode processing performed by the identification system according to Embodiment 2 described above, a description thereof is omitted.

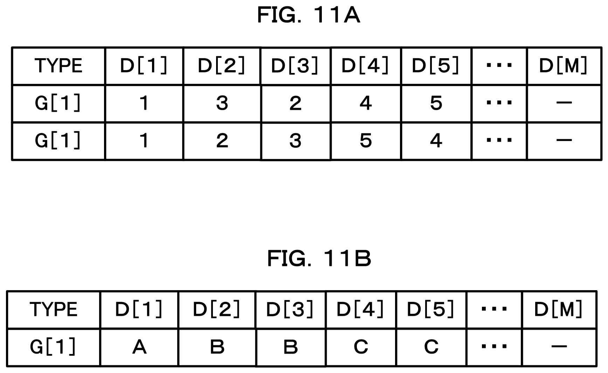

When subsequently, it is determined that a variable m is M (step S8; Yes), a generator 22 of the identifier 11 determines whether or not there exist detection elements 5 the positions of which in orders of magnitudes of detected values are different between generation sources T the types of which are the same (step S12). For example, when, as illustrated in FIG. 11A, there exist detection elements 5 the consecutive positions of which in the orders of the plurality of detection elements 5 change with respect to the generation sources T the types of which are the same (G[1]), the determination in step S12 results in affirmation.

When there exist no detection elements 5 the positions of which in the orders of magnitudes of detection values detected by the plurality of detection elements 5 are replaced with each other or are the same with respect to the generation sources T the types of which are the same (G[1]) (step S12; No), the identifier 11 terminates the registration mode processing.

In contrast, when regarding the generation sources T of the same type (G[1]), there exist, among the plurality of detection elements 5, detection elements 5 the positions of which in the orders of magnitudes of detection values change with respect to the generation sources T the types of which are the same (G[1]) (step S12; Yes), the generator 22 groups detection elements 5 the positions of which in the orders are consecutive among the detection elements the positions of which in the orders change (step S17).

For example, when as illustrated in FIG. 11A, positions of detection elements 5 in the orders of magnitudes of detection values are different between the generation sources T the types of which are the same (type G[1]), the generator 22 of the identifier 11 integrates detection elements 5 the positions of which in the orders are consecutive among the detection elements 5 the positions of which in the orders change into a group A, B, or C, as illustrated in FIG. 11B. Note that information A, B, or C to identify a group can be set by an operation input by a user. For example, A, B, and C can be defined as 1, 2, and 3, respectively.

After processing in step S17 is completed, the generator 22 determines whether or not, even when detection elements 5 are grouped, orders of magnitudes of detection values are different from one another among the generation sources T and each generation source T can be discriminated from the other generation sources T (step S18). In the case where even when detection elements 5 are grouped, the orders of magnitudes of detection values are different among the generation sources T (step S18; Yes), the generator 22 updates order information of the grouped detection elements 5 (step S15) and registers the updated order information (step S7). Subsequently, the identifier 11 terminates the registration mode processing.

Note that although in the case where when detection elements 5 are grouped, some orders of magnitudes of detection values overlap each other and a generation source T cannot be discriminated from the other generation sources T (step S18; No), the generator 22 performs grouping again (step S17), the generator 22 repeats the grouping by changing a threshold value for performing grouping of detection elements 5 (the threshold value is the same as the threshold value for the above-described percentage of correct identifications) until orders that overlap each other are eliminated (step S18; Yes).

As described above, in the present embodiment, in the case where when detection using the plurality of detection elements 5 is performed multiple times with respect to known generation sources the types of which are the same, there exist detection element 5 the positions of which in orders of magnitudes of detection values change and, even when such detection elements 5 are grouped, orders of the grouped detection elements 5 are different from each other between the generation sources T and each generation source T can be discriminated from the other generation sources T, the generator 22 groups detection elements 5 the positions of which in the orders are consecutive among the detection elements 5 the positions of which in the orders change and generates information indicating an order of groups of detection elements 5.

A specifier 21 of the identifier 11 identifies, based on the information indicating an order of groups of detection elements 5 when substances are detected by the detection elements 5 in the groups generated by the generator 22, a type of a generation source T of the substances. Identification mode processing performed by the identifier 11 is the same as the identification mode processing performed by the identification system 1 according to Embodiment 1 described above (see FIG. 7). That is, detection preparation is performed with respect to a generation source T the type of which is unknown (step S21), detection of substances by the plurality of detection elements 5 is performed (step S22), order information is generated (step S23), and through matching of the order information, the type of the generation source T is specified (step S24).

As described in the foregoing, according to the identification system 1 according to the present embodiment, in the case where detection is performed multiple times with respect to generation sources T the types of which are the same, there exist detection elements 5 the positions of which in orders change in the detection results, and even when detection elements 5 the positions of which in the orders are consecutive among the detection elements 5 the positions of which in the orders change are grouped, there exist no orders of groups of detection elements 5 that are the same with respect to generation sources T the types of which are different, the detection elements 5 are grouped. Because of this configuration, it becomes possible to reduce the number of detection elements 5 to be actually used for the identification. This is because one detection element 5 is sufficient for the same group. The identifier 11 identifies, based on information indicating groups of detection elements 5 when substances are detected by the detection elements 5 grouped by the generator 22, a type of a generation source T of the substances.

### Embodiment 4

Next, Embodiment 4 of the present disclosure is described. A configuration of an identification system 1 according to the present embodiment is the same as the configuration of the identification system 1 according to Embodiments 1, 2, and 3 described above illustrated in FIG. 1. Operation, in particular registration mode processing, of an identifier 11 of the identification system 1 according to the present embodiment is different from that of the identification system 1 according to Embodiment 2 described above (see FIG. 8). In the registration mode processing in the present embodiment, grouping of generation sources T is performed.

### Registration Mode Processing

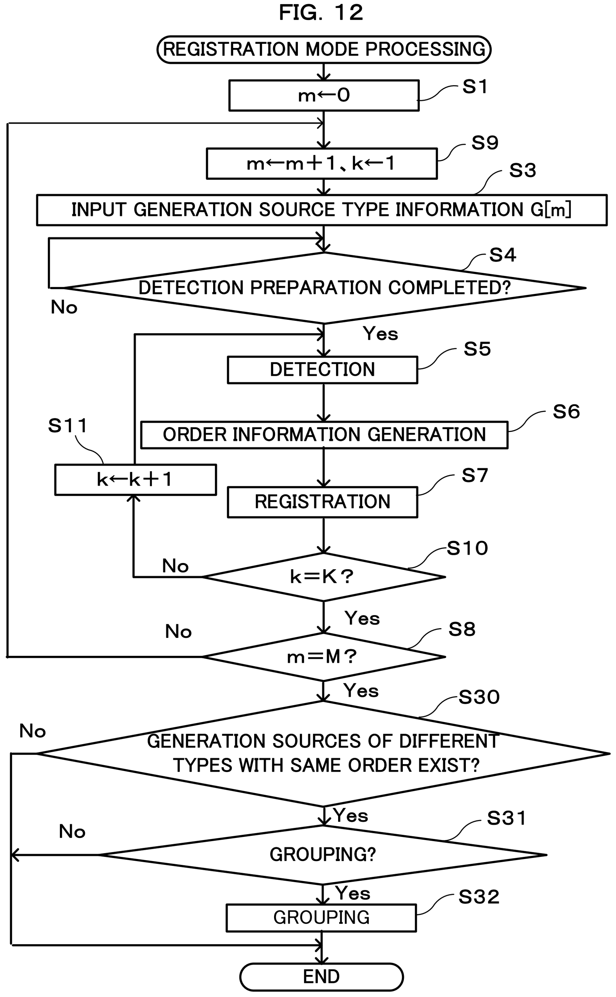

Since, as illustrated in FIG. 12, in the registration mode processing, a process flow in steps S1, S9, S3 to S7, S10, S11, and S8 is the same as that in the registration mode processing performed by the identification system according to Embodiment 2 described above, a description thereof is omitted.

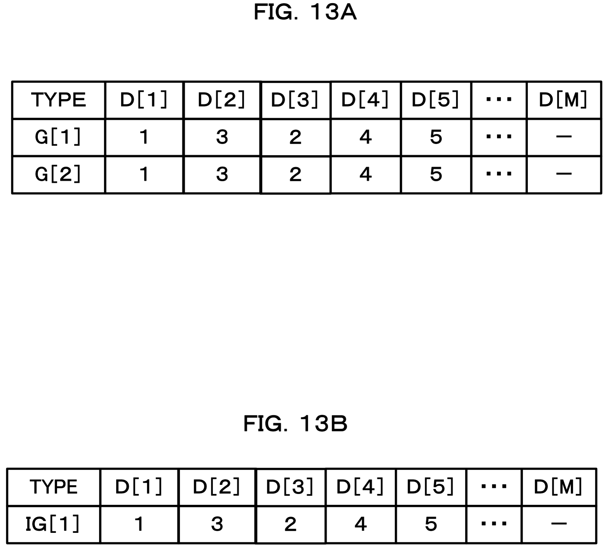

When subsequently, it is determined that a variable m is M (step S8; Yes), a generator 22 of the identifier 11 determines whether or not there exist generation sources T with respect to which orders of magnitudes of detection values detected by a plurality of detection elements 5 are the same and the types of which are different from each other (step S30). For example, when, as illustrated in FIG. 13A, there exist orders of magnitudes of detection values detected by the plurality of detection elements 5 that are the same with respect to a generation source T (type G[1]) and a generation source T (type G[2]), the determination in step S30 results in affirmation.

When there exist no generation sources T with respect to which orders of the detection elements 5 are the same and the types of which are different from each other (step S30; No), the identifier 11 terminates the registration mode processing.

In contrast, when there exist generation sources T with respect to which orders of magnitudes of detection values detected by the detection elements 5 are the same and the types of which are different from each other (step S30; Yes), the generator 22 determines whether or not to perform grouping of the generation sources T (step S31). Whether or not to perform grouping may be set in advance.

When the grouping is not performed (step S31; No), the identifier 11 terminates the registration mode processing. In contrast, when the grouping is performed (step S31; Yes), the generator 22 of the identifier 11 performs the grouping (step S32). For example, when, as illustrated in FIG. 13A, orders of magnitudes of detection values detected by the detection elements 5 are the same between the generation source T (type G[1]) and the generation source T (type G[2]), the generator 22 integrates both orders into one record as a group IG[1], as illustrated in FIG. 13B.

The group IG[1] can be set by an operation input by a user. For example, when G[1] is "tangerine" and G[2] is "grapefruit", the group IG[1] can be set as "citrus fruits". The registered G[1] and G[2] are deleted.

After the processing in step S32 is completed, the identifier 11 terminates the registration mode processing. As described above, the generator 22 groups generation sources T with respect to which orders of the detection elements 5 are the same when detection using the plurality of detection elements 5 is performed with respect to a plurality of known generation sources the types of which are different from one another and generates information indicating an order of the detection elements 5 in association with the group.

A specifier 21 of the identifier 11 identifies, based on information indicating an order of the plurality of detection elements 5 when substances are detected by the plurality of detection elements 5, a type of a generation source T of the substances with reference to information indicating relationships between orders of the plurality of detection elements 5 and types of groups of known generation sources T that is generated by the generator 22. Identification mode processing performed by the identifier 11 is the same as the identification mode processing performed by the identification system 1 according to Embodiment 1 described above (see FIG. 7). That is, detection preparation is performed with respect to a generation source T the type of which is unknown (step S21), detection of substances by the plurality of detection elements 5 is performed (step S22), order information is generated (step S23), and through matching of the order information, the type of the generation source T is specified (step S24).

As described in the foregoing, according to the identification system 1 according to the present embodiment, when there exist orders of the detection elements 5 that are the same with respect to generation sources T the types of which are different from each other, the generation sources T the types of which are different from each other can be integrated into one group. Because of this configuration, when, for example, a new type of tangerine is identified as an unknown generation source T, even when the new type of tangerine cannot be identified as a tangerine, there is a possibility that the new type of tangerine can be identified as a citrus fruit.

### Embodiment 5

Next, Embodiment 5 of the present disclosure is described. A configuration of an identification system 1 according to the present embodiment is the same as the configuration of the identification system 1 according to Embodiment 1 described above illustrated in FIG. 1 except information stored in a storage device 3. In addition, operation, in particular registration mode processing, of an identifier 11 of the identification system 1 according to the present embodiment is different from that of the identification system 1 according to Embodiment 1 described above (see FIG. 6).

The identification system 1 according to the present embodiment identifies, as specific information, a state of a generation source T in place of a type of the generation source T. For example, when a generation source T is a fruit, a fruit changes from an early ripe state, through a fully ripe state, to a rotten state. The identification system 1 according to the present embodiment identifies a state, for example, temporal change, of a generation source T.

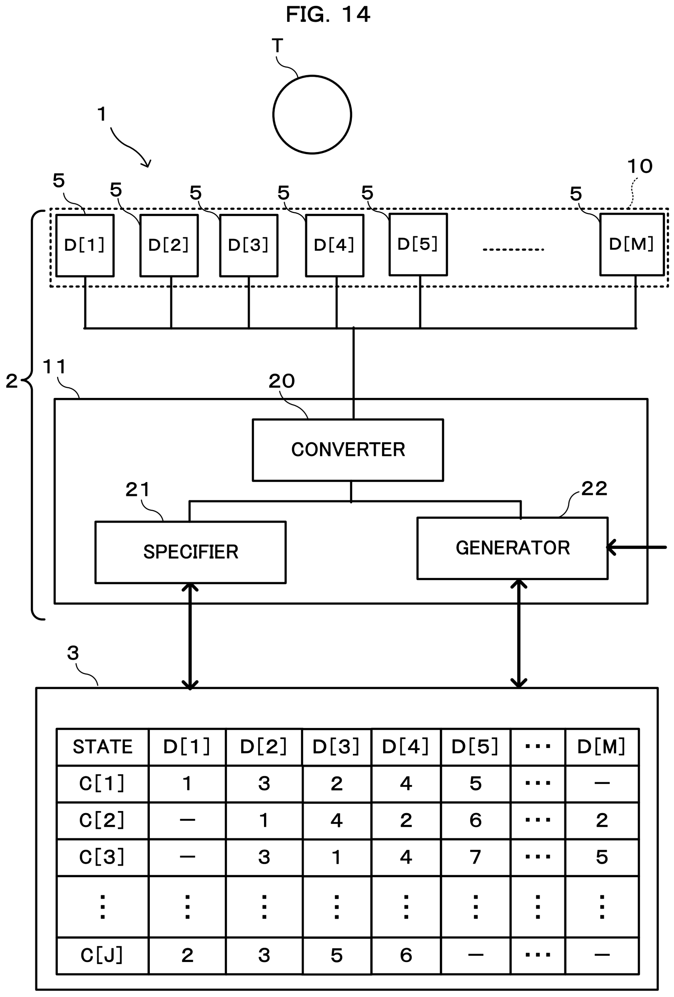

It is now assumed that states of the generation source T are denoted by C[1], C[2], C[3], ... , and C[J]. As illustrated in FIG. 14, in the storage device 3, information indicating an order of a plurality of detection elements 5 based on magnitudes of detection values is stored with respect to each of the states C[1], C[2], C[3], ... , and C[J] of the generation source T. In the storage device 3, the above-described information is stored by registration mode processing executed by a generator 22 of the identifier 11.

### Registration Mode Processing

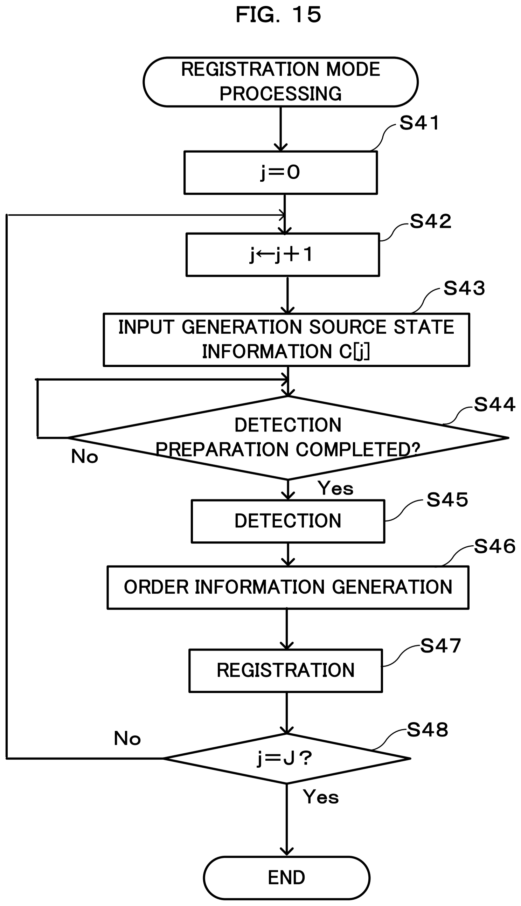

The registration mode processing that is executed by the identifier 11 is described. In the registration mode, j is used as a variable. The variable j takes a value from 1 to J. This value is a value indicating a state of a generation source that is to be identified at a current time. In the registration mode processing, first, in the identifier 11, a converter 20 initializes the variable j to 0 (step S41) and increments the variable j by 1 (step S42), as illustrated in FIG. 15.

Next, the generator 22 constituting the identifier 11 accepts, as input, information indicating a state of a generation source T, that is, generation source state information (step S43). In this step, for example, the state of the generation source T being C[1] is input. Next, the identifier 11 waits until a state in which a generation source T the state of which is known can be detected is established, that is, until detection preparation is completed (step S44; No). In this step, the identifier 11 waits until preparation for detecting C[1] as a state of the generation source T is completed.

When the detection preparation is completed (step S44; Yes), the identifier 11 causes the plurality of detection elements 5 to detect substances emitted from the generation source T (state C[1]) (step S45). Through this processing, from each of the detection elements 5, data including a detection value and identification information of the detection element 5 are sent to the converter 20 of the identifier 11.

When the data are input from the detection elements 5, the converter 20 generates information indicating an order of the plurality of detection elements 5, based on magnitudes of the detection values output from the plurality of detection elements 5 (step S46). Through this processing, for example, information indicating an order of the plurality of detection elements 5, such as D[1], D[3], D[2], D[4], and D[5] in descending order of the detection values, is generated with respect to the generation source T (state C[1]).

Next, the generator 22 of the identifier 11 registers, in the storage device 3, the order generated by the converter 20 and the input information indicating the state of the generation source T in association with each other (step S47).

Next, the generator 22 determines whether or not the variable j is J (step S48). When the variable j is not J (step S48; No), the identifier 11 returns to step S42. Subsequently, the setting of j to 2 (step S42), the input of information about the generation source T (state C[2]) (step S43), the waiting for completion of detection preparation (step S44), the detection (step S45), the order information generation (step S46), the registration (step S47), and the determination of the variable j (step S48) are performed, and further, orders of the plurality of detection elements 5 are generated with respect to the states C[j] (j = 3 to J) of the generation source T and information indicating the orders is stored in the storage device 3 in association with the states C[3] to C[J] of the generation source T. When the variable j is determined to be J (step S48; Yes), the identifier 11 terminates the registration mode processing.

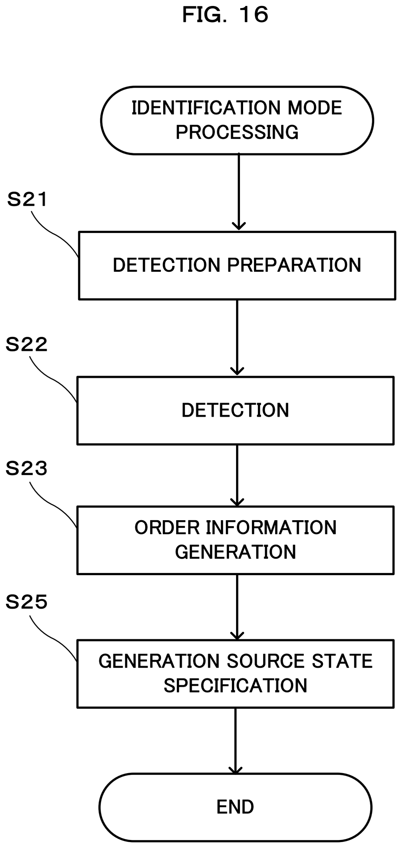

As illustrated in FIG. 16, identification mode processing according to the present embodiment is the same as the identification mode processing of the identification system 1 according to Embodiment 1 described above in that, detection preparation is performed with respect to a generation source T the state of which is unknown (step S21), detection of substances by the plurality of detection elements 5 is performed (step S22), and order information is generated (step S23). After execution of processing in step S23, the generator 22 of the identifier 11 matches the order information with the order information stored in the storage device 3 and specifies a state of the generation source T (step S25).

As described in the foregoing, according to the identification system 1 according to the present embodiment, the state of a generation source T of substances is specified not by detection values, that is, the magnitudes of detection values detected by the plurality of detection elements 5, which are prone to error, but by the order of the magnitudes of the detection values. Since when configured in such a manner, a generation source T can be identified using information that is less likely to contain error, accuracy of identification of a state of a generation source T can be stabilized.

Note that in the identification system 1 according to the present embodiment, as with Embodiments 2 to 4 described above, narrowing down or grouping of the detection elements 5 and grouping of the generation sources T can be performed.

### Embodiment 6

Next, Embodiment 6 of the present disclosure is described. An identification system 1 according to the present embodiment identifies a type or a state of a generation source in consideration of not only an order of a plurality of detection elements 5 but also map data of normalized detection values.

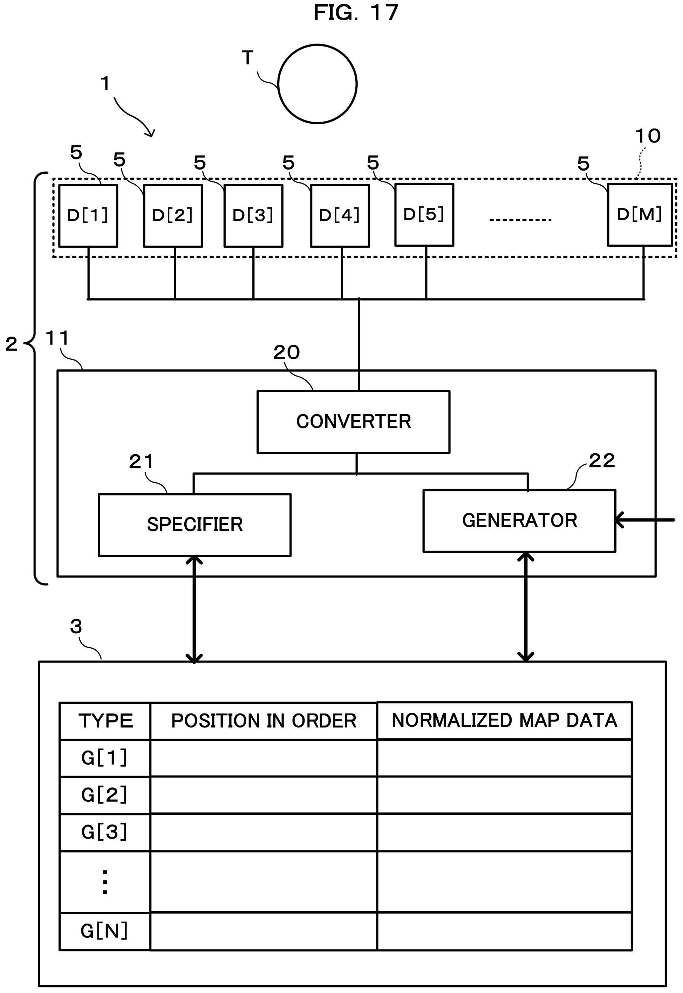

As illustrated in FIG. 17, a configuration of the identification system 1 according to the present embodiment is the same as the configuration of the identification system 1 according to Embodiment 1 described above (see FIG. 1). In the identification system 1, a generator 12 of an identifier 11 generates normalized map data indicating normalized detection values when substances are detected by the plurality of detection elements 5 with respect to each of a plurality of known generation sources T (G[1] to G[N]) the types of which are different from one another, in association with the type of the known generation source and stores the generated normalized map data in a storage device 3. Note that the normalization is performed with a value serving as a reference, such as a maximum value among detection values and a difference between a minimum value and a maximum value, assumed to be 1.

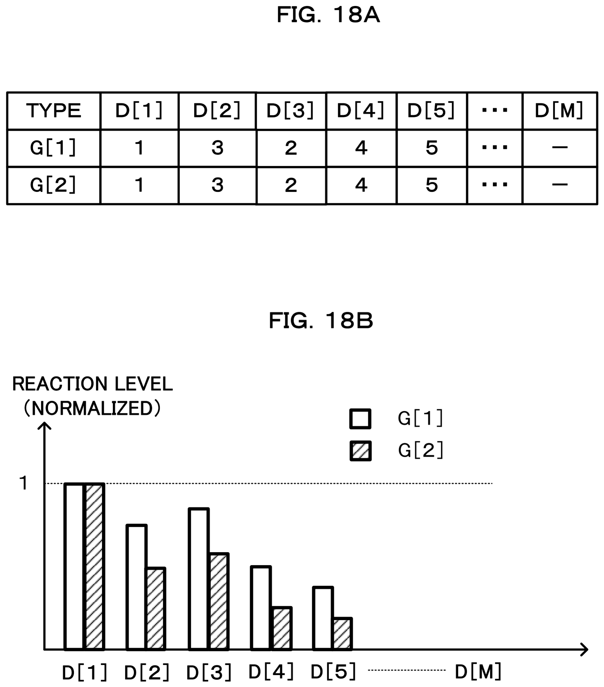

A specifier 21 of the identifier 11 identifies, based on, in conjunction with information indicating an order of detection values when substances are detected by the plurality of detection elements 5, normalized detection values detected by the detection elements 5 when the substances are detected by the plurality of detection elements 5, a type of a generation source T of the substances, with reference to, in conjunction with information indicating relationships between orders of the detection elements 5 and generation sources T the types of which are known, information indicating relationships between normalized detection values detected by the detection elements 5 and the generation sources T the types of which are known. For example, even when, as illustrated in FIG. 18A, orders of magnitudes of detection values detected by the plurality of detection elements 5 are the same between the generation source T (type G[1]) and the generation source T (type G[2]), by matching different pieces of normalized map data as illustrated in FIG. 18B with map data of a generation source T the type of which is unknown, it becomes possible to specify a generation source T having map data the shape of which is closest to (having a high degree of similarity in pattern matching to) the map data of the generation source T the type of which is unknown.

Note that it is also possible to apply the processing performed in consideration of not only an order of magnitudes of detection values detected by the plurality of detection elements 5 but also normalized map data to identification of a state of a generation source T. In addition, it is possible to use map data based on detection values that are not normalized.

### Embodiment 7

Next, Embodiment 7 of the present disclosure is described. In the present embodiment, a detailed configuration of a detector 50 corresponding to a detector 10 constituting an identification system 1 is described.

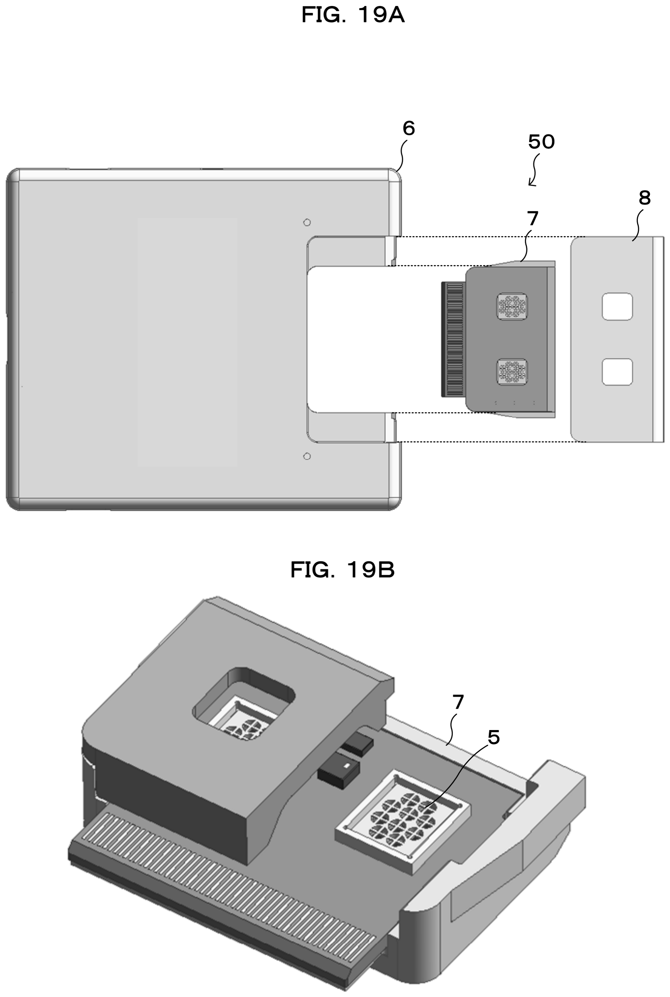

As illustrated in FIG. 19A, the detector 50 includes a detector body 6, a sensor module 7, and a sensor cover 8. On the detector body 6 configured to be capable of inputting and outputting data with an identifier 11, the sensor module 7 is mounted, and the sensor cover 8 protects the sensor module 7 mounted on the detector body 6.

As illustrated in FIG. 19B, on the sensor module 7, 20 detection elements 5 in total are installed. Each of the detection elements 5 has an oscillator having a shape closing a portion of a through-hole, and a substance adsorption film is placed on the oscillator. Substances that the plurality of detection elements 5 installed on the sensor module 7 can detect are different from one another. In each of the detection elements 5, a corresponding substance is detected, and a detection value detected by the detection element 5 is output to the detector body 6 in conjunction with an identification number of the detection element 5. The detector body 6 outputs data including the detection values and the identification numbers to the identifier 11.

As described above, in the detector 10, the sensor module 7 is replaceable with respect to the detector body 6. Replacing the sensor module 7 enables types of detection elements 5 used for detection to be considerably increased. In addition, it is possible to select a sensor module 7 equipped with detection elements 5 capable of detecting a plurality of substances contained in a generation source T to be identified, mount the selected sensor module 7 on the detector body 6, and use the sensor module 7. Because of this configuration, as with, for example, Embodiment 2 described above, it becomes possible to construct an identification device 2 that has only a detection element 5 required to identify an identification target by excluding a detection element 5 required to identify an identification target. In addition, in Embodiment 3 described above, it becomes possible to construct an identification device 2 that has only grouped detection elements 5.

Note that the identifier 11 can be constructed in a computer that is communicable with the detector 10 via a communication network. The same applies to a storage device 3. The storage device 3 can also be constructed in another server computer that is communicable with the computer. Conversely, it may be configured such that, into the device illustrated in FIG. 19A, the identifier 11 is incorporated and the storage device 3 is further incorporated.

In addition, the identifier 11 is only required to include either a specifier 21 or a generator 22. It may be configured such that a developer of the identification system 1, using an identification device 2 including the generator 22, generates information to be stored in the storage device 3 and the user, using an identification device 2 including the specifier 21, identifies a type or a state of a generation source T the type or the state of which is unknown. That is, the generator 22 does not have to be included in the identifier 11.

It is also possible to identify both the type and the state of a generation source T. It is only necessary to store, in the storage device 3, information indicating an order based on magnitudes of detection values detected by the detection elements 5 with respect to each type and each state of a generation source T.

In addition, even in a case where there exist orders including different positions of detection elements 5 between generation sources T the pieces of characteristic information of which are the same, it is only necessary to store such orders in the storage device 3 as it is. In addition, even in a case where there exist orders including the same positions of the detection elements 5 between generation sources T the pieces of characteristic information of which are different from each other, it is only necessary to store such orders in the storage device 3 as it is. In this case, it is only necessary to determine such generation sources T as an identification result.

A detection element 5 that did not react to a generation source T to be identified can be removed from the detector 10 when identifying an unknown generation source T.

The hardware configuration and the software configuration of the identifier 11 are only an example and can be arbitrarily changed and modified.

A core part performing processing of the identifier 11 that includes the CPU 31, the main memory 32, the external memory 33, the operation acceptor 34, the display 35, the input/output device 36, the communicator 37, the internal bus 38, and the like can be achieved using a general computer system instead of a dedicated system. For example, the identifier 11 that execute the above-described processing may be configured by storing and distributing computer programs for performing the above-described operation in a non-transitory computer-readable recording medium (such as a flexible disk, a CD-ROM, and a DVD-ROM) and installing the computer programs in the computer. In addition, the identifier 11 may also be configured by storing the computer programs in a storage device that a server device on a communication network, such as the Internet, has and a general computer system downloading the computer programs.

When the functions of the identifier 11 are to be achieved through sharing between an operating system (OS) and an application program or collaboration between the OS and the application program, only the application program part may be stored in a non-transitory recording medium or a storage device.

It is also possible to superimpose a computer program on a carrier wave and distribute the computer program via a communication network. For example, the computer program may be posted on a bulletin board system (BBS) on the communication network, and the computer program may be distributed via the network. The above-described processing may be configured to be able to be performed by starting up and executing the distributed computer program in a similar manner to other application programs under the control of the OS.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2023-91233, filed on June 1, 2023, the entire disclosure of which is incorporated by reference herein.

### Industrial Applicability

The present disclosure can be applied to identification of a generation source by detecting substances emitted from the generation source.

### Reference Signs List

- 1: Identification system

- 2: Identification device
- 3: Storage device
- 5: Detection element
- 6: Detector body
- 7: Sensor module
- 8: Sensor cover
- 10: Detector
- 11: Identifier
- 20: Converter
- 21: Specifier
- 22: Generator
- 31: CPU
- 32: Main memory
- 33: External memory
- 34: Operation acceptor
- 35: Display
- 36: Input/output device
- 37: Communicator
- 38: Internal bus
- 39: Program
- 50: Detector
- T: Generation source

## Claims

1. An identification device, comprising:
a plurality of detection elements each of which reacts to one of substances of types different from one another among a plurality of types of substances emitted from a generation source and outputs a detection value having a magnitude corresponding to an amount of a substance to which the detection element has reacted; and
an identifier that identifies, based on information indicating an order of the plurality of detection elements when substances are detected by the plurality of detection elements, characteristic information of a generation source of the substances with reference to information indicating a relationship between an order of the plurality of detection elements, the order being ordered based on magnitudes of detection values detected by the plurality of detection elements when substances emitted from a generation source the characteristic information of which indicating a type or a state is known are detected by the plurality of detection elements, and the characteristic information of the known generation source.

2. The identification device according to claim 1, wherein in the substances, a substance causing an odor to be sensed is included.

3. An identification system, comprising:
a plurality of detection elements each of which reacts to one of substances of types different from one another among a plurality of types of substances emitted from a generation source and outputs a detection value having a magnitude corresponding to an amount of a substance to which the detection element has reacted;
a generator that generates information indicating orders of the plurality of detection elements, each of the orders being ordered based on magnitudes of detection values when detection is performed by the plurality of detection elements with respect to one of a plurality of known generation sources pieces of characteristic information of which indicating types or states are different from one another, in association with the pieces of characteristic information of the known generation sources; and
an identifier that identifies, based on information indicating an order of the plurality of detection elements when substances are detected by the plurality of detection elements, the characteristic information of a generation source of the substances with reference to information indicating relationships between orders of the plurality of detection elements and the pieces of characteristic information of the known generation sources, the information being generated by the generator.

4. The identification system according to claim 3, wherein
in a case where when detection using the plurality of detection elements is performed multiple times with respect to known generation sources the pieces of characteristic information of which are same, there exist a detection element a position of which in an order of magnitudes of detection values changes and, even when the detection element is excluded, orders of remaining detection elements do not overlap each other between the generation sources, the generator narrows down the detection elements to the remaining detection elements and generates information indicating an order of the detection elements, and
the identifier identifies, based on information indicating an order of the detection elements when substances are detected by the detection elements that are narrowed down by the generator, the characteristic information of a generation source of the substances.

5. The identification system according to claim 3, wherein
in a case where when detection using the plurality of detection elements is performed multiple times with respect to known generation sources the pieces of characteristic information of which are the same, there exist detection elements positions of which in an order of magnitudes of detection values change and, even when the detection elements are grouped, orders do not overlap each other between generation sources, the generator groups detection elements the positions of which in an order are consecutive among detection elements the positions of which in an order change and generates information indicating an order of groups of the detection elements, and
the identifier identifies, based on information indicating an order of the detection elements when substances are detected by the detection elements in groups generated by the generator, the characteristic information of a generation source of the substances.

6. The identification system according to claim 3, wherein
the generator groups generation sources with respect to which orders of the detection elements are same when detection using the plurality of detection elements is performed with respect to a plurality of known generation sources the pieces of characteristic information of which are different from one another and generates information indicating an order of the detection elements in association with a group, and
the identifier identifies, based on information indicating an order of the plurality of detection elements when substances are detected by the plurality of detection elements, the characteristic information of a group of generation sources of the substances with reference to information indicating a relationship between an order of the plurality of detection elements and a group of the known generation sources, the information being generated by the generator.

7. The identification system according to claim 3, wherein
the generator generates information indicating detection values when detection is performed by the plurality of detection elements with respect to each of a plurality of known generation sources the pieces of characteristic information of which are different from one another in association with the pieces of characteristic information of the known generation sources, and
the identifier identifies, based on, in conjunction with information indicating an order of the detection elements when substances are detected by the plurality of detection elements, detection values detected by the detection elements when substances are detected by the plurality of detection elements, the characteristic information of a generation source of the substances with reference to, in conjunction with information indicating a relationship between an order of the detection elements and the characteristic information of the known generation source, information indicating a relationship between detection values detected by the detection elements and the characteristic information of the known generation source, the information being generated by the generator.

8. An identification method executed by an identification device that identifies a generation source, using a plurality of detection elements each of which reacts to one of substances of types different from one another among a plurality of types of substances emitted from a generation source and outputs a detection value having a magnitude corresponding to an amount of a substance to which the detection element has reacted, the identification method comprising
identifying, based on information indicating an order of the plurality of detection elements when substances are detected by the plurality of detection elements, the characteristic information of a generation source of the substances with reference to information indicating relationships between orders of the plurality of detection elements, the orders being ordered based on magnitudes of detection values detected by the plurality of detection elements when substances emitted from known generation sources pieces of characteristic information of which indicating types or states are different from one another are detected by the plurality of detection elements, and the pieces of characteristic information of the known generation sources.
